(19) Europäisches Patentamt
European Patent Office
Office européen des brevets

(11) EP 1 942 103 A1

(12) EUROPEAN PATENT APPLICATION
published in accordance with Art. 153(4) EPC

(43) Date of publication:
09.07.2008 Bulletin 2008/28

(21) Application number: 06810597.2

(22) Date of filing: 27.09.2006

(51) Int Cl.:
*C07C 303/40* (2006.01) *C07B 53/00* (2006.01)
*C07C 209/62* (2006.01) *C07C 211/27* (2006.01)
*C07C 311/16* (2006.01) *C07B 61/00* (2006.01)

(86) International application number:
PCT/JP2006/319106

(87) International publication number:
WO 2007/037242 (05.04.2007 Gazette 2007/14)

(84) Designated Contracting States:
AT BE BG CH CY CZ DE DK EE ES FI FR GB GR HU IE IS IT LI LT LU LV MC NL PL PT RO SE SI SK TR

(30) Priority: 27.09.2005 JP 2005279003

(71) Applicant: Kaneka Corporation
Kita-ku
Osaka-shi, Osaka 530-8288 (JP)

(72) Inventors:
- TANAKA, Tatsuyoshi
Takasago-shi, Hyogo 676-8688 (JP)
- OKURO, Kazumi
Takasago-shi, Hyogo 676-8688 (JP)
- MITSUDA, Masaru
Takasago-shi, Hyogo 676-8688 (JP)

(74) Representative: Vossius & Partner
Siebertstrasse 4
81675 München (DE)

# (54) METHOD FOR PRODUCING OPTICALLY ACTIVE BENZYLAMINE DERIVATIVE

(57) The present invention relates to a method for producing an optically active benzylamine derivative which is useful as an intermediate for pharmaceutical products and the like. In the present invention, an optically active benzylalcohol derivative is reacted with a sulfonylamide derivative in the presence of a phosphine derivative and an azodicarbonyl compound, to obtain an optically active benzylsulfonylamide derivative as a novel compound. Then, the thus-obtained optically active benzylsulfonylamide derivative is reacted with a thiol derivative, thereby producing an optically active benzylamine derivative. According to the present invention, the compound can be easily produced by a simple and short process without racemization.

EP 1 942 103 A1

## Description

TECHNICAL FIELD

The present invention relates to an optically active benzylamine derivative which is an important intermediate in production in the pharmaceutical and agrochemical fields, and a method for producing the same. In more detail, it relates to a method for converting an optically active benzylalcohol derivative to an optically active benzylamine derivative.

BACKGROUND ART

As a method for producing an optically active benzylamine derivative represented by a general formula (7):

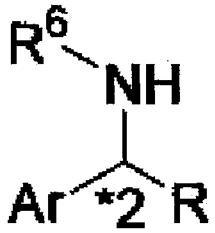

(7)

wherein Ar represents a substituted or unsubstituted aryl group having 6 to 20 carbon atoms or a substituted or unsubstituted heteroaromatic group having 4 to 20 carbon atoms, R represents a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, $R^6$ represents hydrogen, a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, and *2 represents an asymmetric carbon atom, from an optically active benzylalcohol derivative represented by a general formula (1):

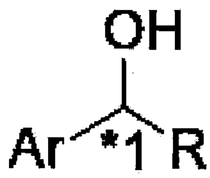

(1)

wherein Ar and R are as defined above, and *1 represents an asymmetric carbon atom, the following methods are reported:

1) a method for producing an optically active benzylamine derivative, in which a hydroxy group in an optically active benzylalcohol derivative is substituted with halogen such as chlorine and then the halogenated benzylalcohol derivative is reacted with a nitrogen nucleophile (Non-Patent Document 1);
2) a method for producing an optically active benzylamine derivative, in which an optically active benzylalcohol derivative is reacted with diphenylphosphoryl azide and then the reactant is reduced (Non-Patent Documents 2 and 3);
3) a method for producing an optically active benzylamine derivative, in which an optically active benzylalcohol derivative is sulfonylated and then the resulting compound is reacted with a nitrogen nucleophile (Patent Document 1);
4) a method for producing an optically active benzylamine derivative, in which an optically active benzylalcohol derivative is reacted with a nitrogen nucleophile in the presence of an azodicarbonyl compound (Non-Patent Document 4); and the like.

Patent Document 1: Japanese Patent Application Laid-Open (JP-A) No. 2002-30048
Non-Patent Document 1: Journal of Organic Chemistry, 1998, 63, 6273
Non-Patent Document 2: Journal of Organic Chemistry, 1996, 61, 3561
Non-Patent Document 3: Tetrahedron Asymmetry, 1996, 7, 2809

Non-Patent Document 4: Journal of Organic Chemistry, 1990, 55, 215

DISCLOSURE OF THE INVENTION

PROBLEMS TO BE SOLVED BY THE INVENTION

However, in the method 1), it has been known that a partial racemization takes place in the neucleophilic substitution reaction at a benzylic position because a SN1 type substitution reaction competes with a SN2 type substitution reaction due to an involvement of a stabilized benzyl cation. Therefore, it is difficult to produce an optically active benzylamine derivative with high optical purity.

In the method 2), because a nitrogen nucleophilic species is azide even though a substitution reaction proceeds in a high asymmetric transfer ratio, in order to produce optically active N-alkylbenzylamine, optically active N-arylbenzylamine, or optically active N-aralkylbenzylamine, it is necessary to perform N-alkylation, N-arylation, or N-aralkylation after reduction of the obtained optically active azide compound. Therefore, the number of steps increases and the operation becomes complicated. Further, it is difficult to obtain an intended substance in a high yield by a monoalkylation reaction of amine because of the generation of byproducts of tertiary amine and/or a quaternary ammonium salt, which is a general problem of the reaction. Furthermore, a handling of an azide compound with an explosion risk is a problematic procedure in terms of both safety and facility in industrial production.

Also, in the method 3), because of nucleophilic substitution at a benzylic position, it is difficult to obtain an optically active benzylamine derivative with high optical purity. The racemization is relatively suppressed in the production method described in Patent Document 1. However, amine that can be produced by the substitution reaction and then deprotection is limited to primary amine because a nitrogen nucleophile that can be used is limited to benzylamine, allylamine, benzylcarbamate, hydroxylamine, phthalimide potassium, sodium azide, and the like. Therefore, in order to synthesize secondary amine, there is a problem that the number of processes increases and the operation becomes complicated, since it is necessary to perform the substitution reaction, a deprotection operation, and then N-alkylation, N-arylation, or N-aralkylation. Further, in the monoalkylation reaction of amine, tertiary amine and/or a quaternary ammonium salt are generally produced as byproducts, and it is difficult to obtain an intended substance in high yield.

In the method 4), it is generally known that a reaction proceeds in a very high asymmetric transfer ratio. However, a nitrogen nucleophile that can be used is limited, and only compounds with low acidity, such as imide, e.g., phthalimide, and diacylamine, can be generally used. In the case of using imide or diacylamine as a nitrogen nucleophile, since the direct synthesis of N-alkyl, N-aryl, or N-aralkylamine is impossible, it is necessary to perform N-alkylation, N-arylation, or N-aralkylation after deprotection, and thus the number of processes increases and the operation becomes complicated. Further, a method of using hydrazine with a high toxicity, or a method of heating under a severe acid condition or alkaline condition is known as a deprotection condition. However, the former has a problem of toxicity, and the latter has a problem of decomposition of a substrate in the case that the substrate has other functional groups.

MEANS FOR SOLVING THE PROBLEMS

In view of the above circumstances, as a result of eager investigation of the reaction of an optically active benzylalcohol derivative with a nitrogen nucleophile with regard to a method for producing an optically active benzylamine, a production method as described below has been found.

That is, the present invention relates to a method for producing an optically active benzylsulfonylamide derivative represented by a general formula (5):

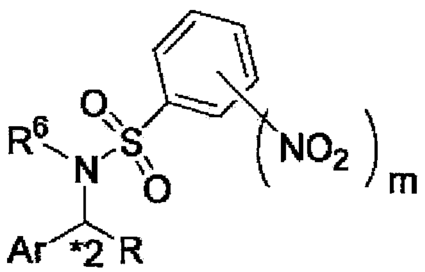

(5)

wherein Ar represents a substituted or unsubstituted aryl group having 6 to 20 carbon atoms or a substituted or unsubstituted heteroaromatic group having 4 to 20 carbon atoms, R represents a substituted or unsubstituted alkyl group

having 1 to 18 carbon atoms, a substituted or unsubstituted aryl group having 6 to 20 carbon atoms, or a substituted or unsubstituted aralkyl group having 7 to 20 carbon atoms, $R^6$ represents hydrogen, a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms,m represents an integer of 1 to 3, and *2 represents an asymmetric carbon atom, characterized by reacting an optically active benzylalcohol derivative represented by a general formula (1):

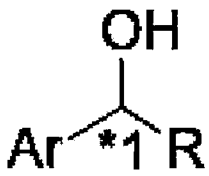

(1)

wherein Ar and R are as defined above, and *1 represents an asymmetric carbon atom, with a sulfonylamide derivative represented by a general formula (4):

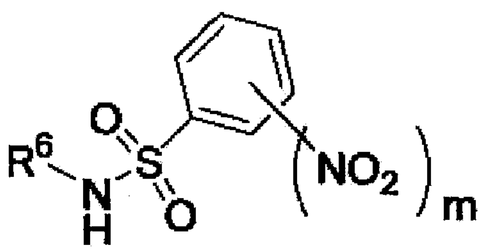

(4)

wherein $R^6$ is as defined above, and m is as defined above.

Further, the present invention relates to a method for producing an optically active benzylamine derivative represented by a general formula (7):

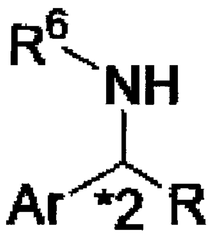

(7)

wherein Ar represents a substituted or unsubstituted aryl group having 6 to 20 carbon atoms or a substituted or unsubstituted heteroaromatic group having 4 to 20 carbon atoms, R represents a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, $R^6$ represents hydrogen, a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, and *2 represents an asymmetric carbon atom, characterized by reacting the optically active benzylsulfonylamide derivative represented by the general formula (5) with a thiol derivative represented by a general formula (6):

$R^7SM$ (6)

wherein $R^7$ represents hydrogen, a substituted or unsubstituted alkyl group having 1 to 18 carbon atoms, a substituted

or unsubstituted aryl group having 6 to 20 carbon atoms, or a substituted or unsubstituted aralkyl group having 7 to 20 carbon atoms, and M represents hydrogen, an alkali metal or an alkaline earth metal.

Further, the present invention relates to the optically active benzylsulfonylamide derivative represented by the general formula (5).

EFFECT OF THE INVENTION

According to the present invention, the production of an optically active benzylamine derivative in a very high asymmetric transfer ratio becomes possible. Further, according to the present invention, optically active N-alkylbenzylamine, optically active N-arylbenzylamine, and optically active N-aralkylbenzylamine are easily prepared by a simple and short process.

BEST MODE FOR CARRYING OUT THE INVENTION

First, a step for producing an optically active benzylsulfonylamide derivative represented by the general formula (5):

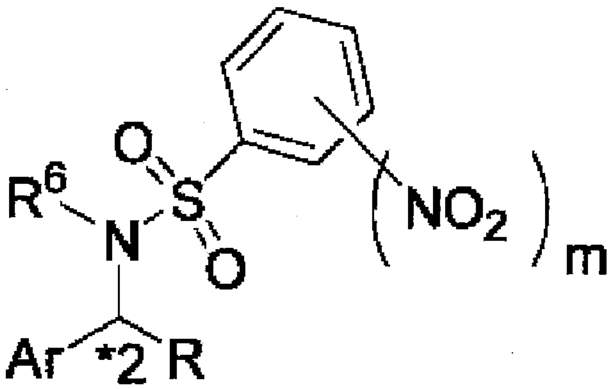

(5)

by reacting an optically active benzylalcohol derivative represented by the general formula (1):

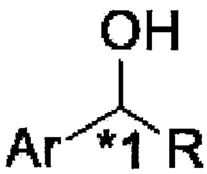

(1)

with an optically active benzylsulfonylamide derivative represented by the general formula (4):

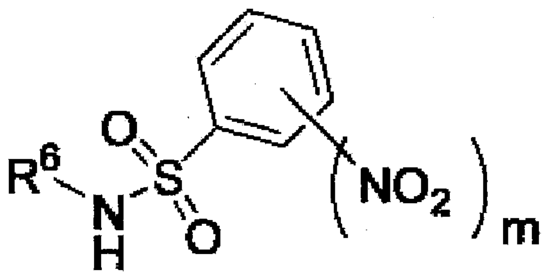

(4)

is described.

In the present description, the number of carbon atoms does not include the number of carbon atoms in substitutional groups. Examples of the substituents of an alkyl group, an aryl group, an aralkyl group, an alkoxy group, an aryloxy group, and an aralkyloxy group, as described below, include an alkyl group, an aryl group, an aralkyl group, an oxy group, an alkoxy group, a nitro group, a thio group, an amino group, a carbonyl group, anitrile group, a sulfonyl group, halogen, and the like. Further, "2-, 3-, or 4-methyl substituted phenyl group" in the present description represents, for example, a 2-methyphenyl group, a 3-methylphenyl group, or a 4-methylphenyl group.

In the general formulae (1) and (5), Ar represents a substituted or unsubstituted aryl group having 6 to 20 carbon atoms or a substituted or unsubstituted heteroaromatic group having 4 to 20 carbon atoms.

Specific examples of the substituted or unsubstituted aryl group having 6 to 20 carbon atoms include alkyl substitutedphenyl groups such as a phenyl group, a 2-, 3-, or 4-methyl substituted phenyl group, a 2,3-, 2,4-, 2,5-, 2,6-, 3,4-, or 3,5-dimethyl substituted phenyl group, a 2,3,4-, 2,3,5-, 2,3,6-, 2,4,5-, 2,4,6-, or 3,4,5-trimethyl substituted phenyl group, a 2-, 3-, or 4-ethyl substituted phenyl group, a 2,3-, 2,4-, 2,5-, 2, 6-, 3,4-, or 3, 5-diethyl substituted phenyl group, a 2, 3,4-, 2,3,5-, 2,3,6-, 2,4,5-, 2,4,6-, or 3,4,5-triethyl substituted phenyl group, a 2-, 3-, or 4-propyl substituted phenyl group, a 2,3-, 2, 4-, 2, 5-, 2, 6-, 3, 4-, or 3, 5-dipropyl substitutedphenyl group, a 2,3,4-, 2,3,5-, 2,3,6-, 2,4,5-, 2,4,6-, or 3,4,5-tripropyl substituted phenyl group, a 2-, 3-, or 4-isopropyl substituted phenyl group, a 2, 3-, 2, 4-, 2, 5-, 2, 6-, 3, 4-, or 3,5-diisopropyl substituted phenyl group, a 2,3,4-, 2,3,5-, 2,3,6-, 2,4,5-, 2,4,6-, or 3,4,5-triisopropyl substituted phenyl group, a 2-, 3-, or 4-butyl substituted phenyl group, a 2,3-, 2,4-, 2,5-, 2, 6-, 3,4-, or 3, 5-dibutyl substituted phenyl group, a 2, 3, 4-, 2,3,5-, 2, 3, 6-, 2, 4, 5-, 2, 4, 6-, or 3, 4, 5-tributyl substituted phenyl group, a 2-, 3-, or 4-isobutyl substituted phenyl group, a 2,3-, 2,4-, 2,5-, 2,6-, 3,4-, or 3,5-diisobutyl substituted phenyl group, a 2,3,4-, 2,3,5-, 2,3,6-, 2,4,5-, 2,4,6-, or 3, 4, 5-triisobutyl substituted phenyl group, a 2-, 3-, or 4-octyl substituted phenyl group, a 2,3-, 2,4-, 2,5-, 2,6-, 3,4-, or 3, 5-dioctyl substituted phenyl group, a 2,3,4-, 2,3,5-, 2,3,6-, 2, 4, 5-, 2, 4, 6-, or 3,4,5-trioctyl substituted phenyl group, a 2-, 3-, or 4-dodecyl substituted phenyl group, a 2,3-, 2,4-, 2,5-, 2,6-, 3,4-, or 3,5-didodecyl substituted phenyl group, a 2,3,4-, 2,3,5-, 2, 3, 6-, 2, 4, 5-, 2,4,6-, or 3, 4, 5-tridodecyl substituted phenyl group, a 2-, 3-, or 4-trifluoromethyl substituted phenyl group, a 2,3-bis(trifluoromethyl) phenyl group, a 2,4-, 2,5-, 2,6-, 3,4-, or 3,5-bis(trifluoromethyl) substituted phenyl group, a2,3,4-tris(trifluoromethyl) phenyl group, a 2,3,5-, 2,3,6-, 2,4,5-, 2,4,6-, or 3, 4, 5-tris (trifluoromethyl) substituted phenyl group, a 2-, 3-, or 4-trifluoroethyl substituted phenyl group, a 2, 3-, 2,4-, 2,5-, 2, 6-, 3, 4-, or 3,5-bis (trifluoroethyl) substituted phenyl group, and a 2,3,4-, 2,3,5-, 2,3,6-, 2,4,5-, 2,4,6-, or 3, 4, 5-tris (trifluoroethyl) substituted phenyl group; an oxygen atom substituted phenyl group such as a 2-, 3-, or 4-hydroxy substituted phenyl group, a 2,3-, 2,4-, 2,5-, 2,6-, 3,4-, or 3,5-dihydroxy substituted phenyl group, and a 2,3,4-, 2, 3, 5-, 2,3,6-, 2,4,5-, 2,4,6-, or 3,4,5-trihydroxy substituted phenyl group, a 2-, 3-, or 4-methoxy substituted phenyl group, a 2,3-dimethoxy phenyl group, a 2,4-, 2,5-, 2,6-, 3,4-, or 3,5-dimethoxy substituted phenyl group, and a 2, 3, 4-, 2, 3, 5-, 2, 3, 6-, 2, 4, 5-, 2, 4, 6-, or 3, 4, 5-trimethoxy substituted phenyl group, a 2-, 3-, or 4-ethoxy substituted phenyl group, a 2,3-, 2, 4-, 2,5-, 2, 6-, 3,4-, or 3, 5-diethoxy substitutedphenyl group, and a 2,3,4-,2,3,5-, 2,3,6-, 2,4,5-, 2,4,6-, or 3,4,5-triethoxy substituted phenyl group, a 2-, 3-, or 4-butoxy substituted phenyl group, a 2,3-, 2,4-, 2,5-, 2,6-, 3,4-, or 3,5-dibutoxy substituted phenyl group, and a 2,3,4-, 2,3,5-, 2,3,6-, 2,4,5-, 2,4,6-, or 3, 4, 5-tributoxy substituted phenyl group, a 2-, 3-, or 4-octoxy substituted phenyl group, a 2, 3-, 2, 4-, 2,5-, 2,6-, 3,4-, or 3,5-dioctoxy substituted phenyl group, and a 2,3,4-, 2,3,5-, 2,3,6-, 2,4,5-, 2,4,6-, or 3,4,5-trioctoxy substituted phenyl group, a 2-, 3-, or 4-trifluoromethoxy substituted phenyl group, a 2,3-, 2,4-, 2, 5-, 2,6-, 3,4-, or 3,5-bis(trifluoromethoxy) substituted phenyl group, and a 2,3,4-, 2,3,5-, 2,3,6-, 2,4,5-, 2,4,6-, or 3,4,5-tris (trifluoromethoxy) substituted phenyl group; a sulfur substituted phenyl group such as a 2-, 3-, or 4-(methylthio) substituted phenyl group, a 2,3-, 2,4-, 2,5-, 2,6-, 3,4-, or 3,5-bis(methylthio) substituted phenyl group, and a 2,3,4-, 2,3,5-, 2,3,6-, 2,4,5-, 2,4,6-, or 3, 4, 5-tris (methylthio) substituted phenyl group, a 2-, 3-, or 4- (ethylthio) substitutedphenyl group, a 2,3-, 2,4-, 2,5-, 2,6-, 3,4-, or 3,5-bis(ethylthio) substituted phenyl group, and a 2,3,4-, 2,3,5-, 2,3,6-, 2,4,5-, 2,4,6-, or 3,4,5-tris(ethylthio) substituted phenyl group, a 2-, 3-, or 4- (octylthio) substitutedphenyl group, a 2, 3-, 2,4-, 2,5-, 2,6-, 3,4-, or 3,5-bis(octylthio) substituted phenyl group, and a 2,3,4-, 2,3,5-, 2,3,6-, 2,4,5-, 2,4,6-, or 3,4,5-tris(octylthio)

substituted phenyl group; an amino substitutedphenyl group such as a 2-, 3-, or 4-amino substituted phenyl group, a 2,3-, 2,4-, 2,5-, 2,6-, 3,4-, or 3,5-diamino substituted phenyl group, and a 2,3,4-, 2,3,5-, 2,3,6-, 2,4,5-, 2, 4, 6-, or 3, 4, 5-triamino substituted phenyl group, a 2-, 3-, or 4-(N-methylamino) substituted phenyl group, a 2,3-, 2,4-, 2, 5-, 2, 6-, 3,4-, or 3,5-bis (N-methylamino) substituted phenyl group, and a 2,3,4-, 2,3,5-, 2,3,6-, 2,4,5-, 2,4,6-, or 3, 4, 5-tris (N-methylamino) substituted phenyl group, a 2-, 3-, or 4- (N-ethylamino) substituted phenyl group, a 2,3-, 2, 4-, 2,5-, 2,6-, 3, 4-, or 3,5-bis (N-ethylamino) substituted phenyl group, and a 2,3,4-, 2,3,5-, 2,3,6-, 2,4,5-, 2,4,6-, or 3,4,5-tris(N-ethylamino) substituted phenyl group, a 2-, 3-, or 4- (N,N-dimethylamino) substituted phenyl group, a 2,3-, 2,4-, 2,5-, 2,6-, 3,4-, or 3,5-bis(N,N-dimethylamino) substituted phenyl group, and a 2, 3, 4-, 2, 3, 5-, 2, 3, 6-, 2, 4, 5-, 2, 4, 6-, or 3,4,5-tris(N,N-dimethylamino) substituted phenyl group, a 2-, 3-, or 4-(N,N-dibutylamino) substituted phenyl group, a 2,3-, 2,4-, 2,5-, 2,6-, 3,4-, or 3,5-bis(N,N-dibutylamino) substituted phenyl group, and a 2,3,4-, 2,3,5-, 2,3,6-, 2,4,5-, 2,4,6-, or 3,4,5-tris(N,N-dibutylamino) substituted phenyl group; a halogen substituted phenyl group such as a 2-, 3-, or 4-chloro-substituted phenyl group, a 2,3-, 2,4-, 2,5-, 2,6-, 3,4-, or 3,5-dichloro-substituted phenyl group, and a 2, 3, 4-, 2,3,5-, 2,3,6-, 2,4,5-, 2,4,6-, or 3,4,5-trichloro-substituted phenyl group, a 2-, 3-, or 4-bromo-substituted phenyl group, a 2,3-, 2, 4-, 2, 5-, 2, 6-, 3, 4-, or 3, 5-dibromo-substitutedphenyl group, and a 2,3,4-, 2,3,5-, 2,3,6-, 2, 4, 5-, 2,4,6-, or 3,4,5-tribromo-substituted phenyl group, a 2-, 3-, or 4-fluoro-substituted phenyl group, a 2,3-, 2,4-, 2,5-, 2,6-, 3,4-, or 3,5-difluoro-substituted phenyl group, and a 2,3,4-, 2,3,5-, 2,3,6-, 2,4,5-, 2,4,6-, or 3,4,5-trifluoro-substituted phenyl group; and an aryl substituted phenyl group such as a 2-, 3-, or 4-phenyl substituted phenyl group, a 2,3-, 2,4-, 2,5-, 2,6-, 3,4-, or 3,5-diphenyl substituted phenyl group, and a 2,3,4-, 2,3,5-, 2,3,6-, 2,4,5-, 2,4,6-, or 3,4,5-triphenyl substituted phenyl group, a 2-, 3-, or 4-(2-chlorophenyl) substituted phenyl group, a 2,3-, 2,4-, 2,5-, 2,6-, 3,4-, or 3,5-bis (2-chlorophenyl) substituted phenyl group, and a 2, 3, 4-, 2,3,5-, 2, 3, 6-, 2, 4, 5-, 2, 4, 6-, or 3, 4, 5-tris (2-chlorophenyl) substituted phenyl group.

Specific examples of the substituted or unsubstituted heteroaromatic group include a 2-furyl group, a 3-furyl group, a 3-, 4-, or 5-methyl substituted-2-furyl group, a 2-, 4-, or 5-methyl substituted-3-furyl group, a 3-, or 5-ethyl substituted-2-furyl group, a 3-, or 5-phenyl substituted-2-furyl group,a2-thienylgroup,a3-thienyl group, a 3-, 4-, or 5-methyl substituted-2-thienyl group, a 2-, 4-, or 5-methyl substituted-3-thienyl group, a 3-, or 5-ethyl substituted-2-thienyl group, a 3-, or 5-phenyl substituted-2-thienyl group, a 2-pyrrolyl group, a 3-pyrrolyl group, a 3-, 4-, or 5-methyl substituted-2-pyrrolyl group, a 2-, 4-, or 5-methyl substituted-3-pyrrolyl group, a 3-, or 5-ethyl substituted-2-pyrrolyl group, a 3-, or 5-phenyl substituted-2-pyrrolyl group, a 2-pyridyl group, a 3-pyridyl group, a 4-pyridyl group, a 3-, 4-, 5- or 6-methyl substituted-2-pyridyl group, a 2-, 4-, 5-, or 6-methyl substituted-3-pyridyl group, a 3-, or 5-ethyl substituted-2-pyridyl group, a 3-, or 5-phenyl substituted-2-pyridyl group, a 2-imidazole group, a 4- or 5-methyl substituted-2-imidazole group, a 3-isothiazolyl group, a 4-isothiazolyl group, a 5-isothiazolyl group, a 4- or 5-methyl substituted-3-isothiazolyl group, a 3-methyl-4-isothiazolyl group, a 5-phenyl-3-isothiazolyl group, a 2-indoyl group, a 3-indoyl group, a 4-methyl-2-indoyl group, and a 7-methyl-4-indoyl group.

Among these, Ar is preferably a substituted or unsubstituted aryl group having 6 to 20 carbon atoms, more preferably a phenyl group substituted with one to three trifluoromethyl groups, fluoro groups, or trifluoromethoxy groups, and especially preferably a phenyl group substituted with one to three trifluoromethyl groups. Specific examples include a 2-, 3-, or 4-trifluoromethyl substituted phenyl group, a 2,3-, 2,4-, 2,5-, 2,6-, 3,4-, or 3,5-bis(trifluoromethyl) substituted phenyl group, and a 2,3,4-, 2,3,5-, 2,3,6-, 2,4,5-, 2,4,6-, or 3,4,5-tris(trifluoromethyl) substituted phenyl group, and more preferably a 3,5-bis(trifluoromethyl) phenyl group.

In the general formulae (1) and (5), R represents a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms.

Examples of the substituted or unsubstituted alkyl group having 1 to 18 carbon atoms include a linear alkyl group such as a methyl group, an ethyl group, an n-propyl group, an isopropyl group, an n-butyl group, an isobutyl group, an s-butyl group, a t-butyl group, an n-penthyl group, an isopenthyl group, an n-hexyl group, an n-octyl group, an n-dodecyl group, and a t-dodecyl group, and a cyclic alkyl group such as a cyclopropyl group, a cyclobutyl group, a cyclopenthyl group, and a cyclohexyl group.

Examples of the substituted or unsubstituted aryl group having 6 to 20 carbon atoms include, for example, a phenyl group, a 1-naphthyl group, a 2-naphthyl group, a 2-methylphenyl group, a 3-methylphenyl group, a 4-methylphenyl group, a 2-ethylphenyl group, a 3-ethylphenyl group, a 4-ethylphenyl group, a 2-methoxyphenyl group, a 3-methoxyphenyl group, a 4-methoxyphenyl group, a 2-nitrophenyl group, a 4-phenylphenyl group, a 4-chlorophenyl group, a 4-bromophenyl group, a 4-fluorophenyl group, and the like.

Examples of the substituted or unsubstituted aralkyl group having 7 to 20 carbon atoms include , for example, a benzyl group, a 2-methylbenzyl group, a 3-methylbenzyl group, a 4-methylbenzyl group, a 2-methoxybenzyl group, a 3-ethoxybenzyl group, an 1-phenylethyl group, a 2-phenyl ethyl group, a 1-(4-methylphenyl)ethyl group, a 1-(4-methoxyphenyl)ethyl group, a 3-phenylpropyl group, and a 2-phenylpropyl group.

Among these groups, an unsubstituted alkyl group is preferable, and a methyl group is more preferable.

*1 represents an asymmetric carbon atom. Its absolute configuration is not especially limited, and may be a (R) -isomer or an (S)-isomer. However, the absolute configuration is preferably an (S)-isomer. Further, *2 represents an asymmetric carbon atom. Its absolute configuration is not especially limited, and may be a (R)-isomer or an (S)-isomer. However, the absolute configuration is preferably a (R) -isomer. Because the reaction in the present step proceeds with a stereo-inversion, a benzylsulfonylamide derivative of an (S) -isomer is produced from a benzylalcohol derivative of a (R)-isomer, and a benzylsulfonylamide derivative of a (R) -isomer is produced from a benzylalcohol derivative of an (S)-isomer.

In the general formulae (4) and (5), $R^6$ represents hydrogen, a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms. Specific examples of the substituted or unsubstituted alkyl group having 1 to 18 carbon atoms, the substituted or unsubstituted aryl group having 6 to 20 carbon atoms, or the substituted or unsubstituted aralkyl group having 7 to 20 carbon atoms include the groups described above. Among these groups, an unsubstituted alkyl group having 1 to 18 carbon atoms is preferable as $R^6$, and a methyl group and an ethyl group are more preferable.

In the general formulae (4) and (5), m represents an integer of 1 to 3. The substituted position by the nitro group is not especially limited. However, mono substitution at the 2-position, mono substitution at the 3-position, mono substitution at the 4-position, or di substitutions at the 2,4-positions is preferable. Specific examples of nitro group substituted phenyl group are a 2-nitrophenyl group, a 3-nitrophenyl group, a 4-nitrophenyl group, and a 2, 4-dinitrophenyl group. More preferably, m is 1 and the nitro group substituted phenyl group is a 2-nitrophenyl group, a 3-nitrophenyl group, or a 4-nitrophenyl group.

The optically active benzylalcohol derivative (1) can be synthesized easily by asymmetric hydrogenation of the corresponding ketone according to Tetrahedron Asymmetry, 2003, 14, 3581 for example.

A conversion from the optically active benzylalcohol derivative (1) to the optically active benzylsulfonylamide derivative (5) can be performed in the presence of a phosphine derivative represented by a general formula (2):

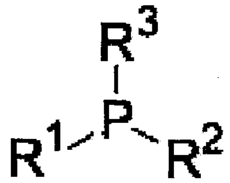

(2)

and an azodicarbonyl compound represented by a general formula (3):

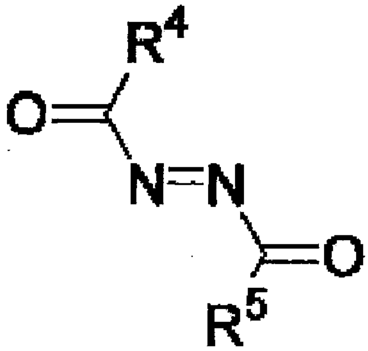

(3)

In the general formula (2), $R^1$, $R^2$, and $R^3$ are independent from each other, and may be the same or different. They represent a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms. Specific examples include the same groups as those exemplified by R. The derivative where all of $R^1$, $R^2$, and $R^3$ are a phenyl group or an n-butyl group is less expensive, low in toxicity, and more preferable.

In a general formula (3), $R^4$ and $R^5$ are independent from each other, and may be the same or different. They represent a substituted or unsubstituted alkoxy group having 1 to 18 carbon atoms, a substituted or unsubstituted aryloxy group having 6 to 20 carbon atoms, a substituted or unsubstituted aralkyloxy group having 7 to 20 carbon atoms, or a substituted or unsubstituted amino group.

Examples of the substituted or unsubstituted alkoxy group having 1 to 18 carbon atoms include a methoxy group, an ethoxy group, an n-propoxy group, an isopropoxy group, an n-butoxy group, an isobutoxy group, an s-butoxy group, a t-butoxy group, an n-pentyloxy group, an isopentyloxy group, an n-hexyloxy group, an n-octyloxy group, ann-dodecyloxy group, a t-dodecyloxy group, and the like.

Examples of the substituted or unsubstituted aryloxy group having 6 to 20 carbon atoms include a phenyloxy group, an 1-naphthyloxy group, a 2-naphthyloxy group, a 2-methylphenyloxy group, a 3-methylphenyloxy group, a 4-methylphenyloxy group, a 2-ethylphenyloxy group, a 3-ethylphenyloxy group, a 4-ethylphenyloxy group, a 2-methoxyphenyloxy group, a 3-methoxyphenyloxy group, a 4-methoxyphenyloxy group, a 2-nitrophenyloxy group, a 4-phenylphenyloxy group, a 4-chlorophenyloxy group, a 4-bromophenyloxy group, a 4-fluorophenyloxy group, and the like.

Example of the substituted or unsubstituted aralkyloxy group having 7 to 20 carbon atoms include a benzyloxy group, a 2-methylbenzyloxy group, a 3-methylbenzyloxy group, a 4-methylbenzyloxy group, a 2-methoxybenzyloxy group, a 3-ethoxybenzyloxy group, a 1-phenylethyloxy group, a 2-pehnylethyloxy group, a 1-(4-methylphenyl)ethyloxy group, a 1- (4-methoxyphenyl) ethyloxy group, a 3-phenylpropyloxy group, a 2-phenylpropyloxy group, and the like.

Examples of the substituted amino group include an amino group in which any of a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms is mono-substituted or di-substituted independently. Specific examples of these substituents of the amino group include the same groups exemplified by R.

Among these, compounds in which both $R^4$ and $R^5$ are preferably a methoxy group, an ethoxy group, an isopropyl group, or a benzyloxy group are preferable because they are industrially easily available. Compound in which both $R^4$ and $R^5$ are an isopropoxy group are more preferable.

The used amount of the phosphine derivative (2) in the present step may be normally 1 equivalent or more to the optically active benzylalcohol derivative (1), preferably 1 to 10 equivalents, and more preferably 1 to 3 equivalents.

The used amount of the azodicarbonyl compound (3) in the present step may be normally 1 equivalent or more to the optically active benzylalcohol derivative (1), preferably 1 to 10 equivalents, and more preferably 1 to 3 equivalents.

The used amount of the sulfonylamide derivative (4) in the present step may be normally 1 equivalent or more to the optically active benzylalcohol derivative (1), preferably 1 to 10 equivalents, and more preferably 1 to 3 equivalents.

In the present step, the solvent used is not particularly limited, and examples thereof include a non-protonic polar solvent such as N,N-dimethylformamide (DMF), dimethylsulfoxide (DMSO), N-methylpyrrolidone, and hexamethylphosphoric triamide; a hydrocarbon solvent such as hexamethylbenzene, toluene, n-hexane, and cyclohexane; an ether solvent such as diethylether, tetrahydrofuran (THF), diisopropylether, methyltert-butylether, and dimethoxyethane; a halogen solvent such as chlorobenzene, methylene chloride, chloroform, and 1,1,1-trichloroethane; an ester solvent such as ethyl acetate and butyl acetate; a nitrile solvent such as acetonitrile and butylonitrile; alcohol such as methanol, ethanol, isopropanol, butanol, and octanol; water, and the like. These may be used alone or two or more kinds may be used in combination. Among these, tetrahydrofuran, toluene, and ethyl acetate are preferable, and toluene is more preferable.

The reaction temperature is normally in the range of -50 to 110°C, and preferably -20 to 50°C.

In the present reaction, the addition order of the benzylalcohol derivative (1), the phosphine derivative (2), the azodicarbonyl compound (3), and the solvent is not especially limited.

A general work-up process may be performed in order to obtain a product from a reaction mixture after the reaction. For example, water is added to the reaction mixture after the reaction and an extraction operation is performed using a general extraction solvent such as ethyl acetate, diethylether, methylene chloride, toluene, and hexane. The reaction solvent and the extraction solvent are distilled off from the obtained extraction liquid by an operation such as heating under reduced pressure, to obtain an intended compound. Further, the reaction solvent is distilled off by an operation such as heating under reduced pressure after the reaction, and then, the similar operation may be performed. In addition, a method, in which a hydrocarbon solvent such as hexane and heptane is added to the reaction mixture, in order to precipitate phosphine oxide produced as a byproduct, and then the precipitated solid is filtered off and the solvent is distilled off, may be performed. The intended compound obtained in such way is nearly pure. However, purity may be increased further by performing a general purification method such as crystallization purification, fractional distillation, and column chromatography.

Next, a step for producing an optically active benzylamine derivative represented by the general formula (7):

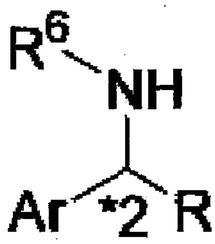

(7)

by reacting the optically active benzylsulfonylamide derivative represented by the general formula (5) with a thiol derivative represented by the general formula (6):

$R^7SM$ (6)

is described.

In the general formula (7), Ar, R, $R^6$, and *2 are as defined above.

In the general formula (6), $R^7$ represents hydrogen, a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms. Specific examples include the same groups exemplified by R. Among these groups, unsubstituted alkyl group and a phenyl group is preferable, and a dodecyl group is more preferable.

Further, M represents hydrogen, an alkali metal such as lithium, sodium, potassium, and cesium, or an alkaline earth metal such as beryllium, magnesium, and calcium. Among these, hydrogen and an alkaline metal are preferable, and hydrogen, sodium, and potassium are more preferable.

The optically active benzylsulfonylamide derivative (5) may be used in a form of the reaction mixture obtained in the above-described process or in an isolated or purified form.

The used amount of the thiol derivative (6) in the present step may be normally 1 equivalent or more to the optically active benzylsulfonylamide derivative (5), preferably 1 to 10 equivalents, and more preferably 1 to 3 equivalents.

In the present step, a base may coexist. In the case that M in the thiol derivative (6) is hydrogen, the reaction is promoted by the coexistence of the base. Examples of the base used include a metal hydroxide such as sodium hydroxide, potassium hydroxide, lithium hydroxide, cesium hydroxide, magnesium hydroxide, and calcium hydroxide, and carbonate such as sodium hydrogen carbonate, sodium carbonate, potassium carbonate, calcium carbonate, and magnesium carbonate. Among these bases, sodium hydroxide, potassium hydroxide, cesium hydroxide, sodium carbonate, and potassium carbonate are preferable, and sodium hydroxide and potassium hydroxide are especially preferable.

These bases may be used without being modified. However, especially in the case of using sodium hydroxide or potassium hydroxide, an aqueous solution of 1 to 50% by weight is preferable, and an aqueous solution of 5 to 30% by weight is more preferable.

The amount of the base used may be normally 1 equivalent or more to the optically active benzylsulfonylamide derivative (5), preferably 1 to 10 equivalents, and more preferably 1 to 5 equivalents.

The reaction temperature is normally in the range of -50 to 110°C, and preferably 0 to 110°C.

In the present step, the solvent used is not especially limited, and specific examples thereof are the same solvents given in the step for producing the optically active benzylsulfonylamide derivative. Among these solvents, dimethylsulfoxiode, N,N-dimethylformamide, acetonitrile, water, and toluene are preferable, and a two-layer system of water-toluene is more preferable.

In the present step, it is preferable that a phase transfer catalyst coexists because a reaction may be promoted. Examples of the phase transfer catalyst used include quaternary ammonium salts such as tetrabutylammonium chloride, tetrabutylammonium bromide, benzyltriethylammonium chloride, benzyltriethylammonium bromide, trioctylmethylammonium chloride, and trioctylmethylammonium bromide, phosphonates such as tetrabutylphosphonium chloride, tetrabutylphosphonium bromide, tetraphenylphosphonium chloride, and tetraphenylphosphonium bromide, crown ether such as 15-crown-5 and 18-crown-6, and polyether such as polyethylene glycol. It is preferably quaternary ammonium salts, and more preferably tetrabutylammonium bromide.

The used amount of the phase transfer catalyst, when it coexists, is preferably 0.001 to 200 mol% to the optically active benzylsulfonylamide derivative, and more preferably 0. 01 to 100 mol%.

The addition order of the substrate, the catalyst, and the solvent in the present reaction is not especially limited.

After the reaction, a general work-up process may be performed in order to obtain a product from the reaction mixture. For example, water is added to the reaction mixture after the reaction and an extraction operation is performed

using a general extraction solvent such as ethylacetate, diethylether, methylene chloride, toluene, and hexane. The reaction solvent and the extraction solvent are distilled off from the obtained extraction liquid by an operation such as a heating under reduced pressure, to obtain an intended compound. Further, the reaction solvent is distilled off by an operation such as a heating under reduced pressure after the reaction, and then, the same operation may be performed. The intended compound obtained in such way is nearly pure. However, purity may be increased further by performing purification by a general method such as crystallization purification, fractional distillation, and column chromatography.

Further, the optically active benzylsulfonylamide derivative (5) that can be produced effectively according to the present invention is a novel compound that has not been described in any documents as an optically active compound. The compound is invented with the above-described production method, and is developed to use as a pharmaceutical intermediate, as a result of the present inventors' investigation.

Ar, R, $R^6$, m, and *2 in the optically active benzylsulfonylamide derivative (5) that is the compound in the present invention are as defined above.

Ar is preferably a phenyl group substituted with one to three trifluoromethyl groups, fluoro groups, or trifluoromethoxy groups, and more preferably a phenyl group substituted with one to three trifluoromethyl groups. Specifically, it is a 2-, 3-, or 4-trifluoromrthyl substituted phenyl group, 2,3-, 2,4-, 2,5-, 2,6-, 3,4-, or 3,5-bis(trifluoromethyl) substituted phenyl group, a 2,3,4-, 2, 3, 5-, 2, 3, 6-, 2, 4, 5-, 2, 4, 6-, or 3, 4, 5-tris (trifluoromethyl) substituted phenyl group. More preferably, it is a 3,5-bis(trifluoromethyl) phenyl group.

R is preferably unsubstituted alkyl group, and more preferably a methyl group.

$R^6$ is preferably unsubstituted alkyl group, and more preferably, a methyl group and an ethyl group.

m is preferably 1 or 2. Mono substitution at the 2-position, mono substitution at the 3-position, mono substitution at the 4-position, and di substitutions at the 2,4-position are more preferable. Specific examples of the nitro-substituted phenyl group are a 2-nitrophenyl group, a 3-nitrophenyl group, a 4-nitrophenyl group, and a 2,4-dinitrophenyl group. More preferably, m is 1, and nitro-substituted phenyl group is a 2-nitrophenyl group, a 3-nitrophenyl group, or a 4-nitrophenyl group.

*2 may be an (R)-isomer or an (S)-isomer. However, it is preferably an (R)-isomer.

## Examples

Hereinafter, the present invention is explained further in detail with reference to Examples. However, the present invention is not limited to these Examples.

(Example 1)

A solution of diisopropylazodicarboxylate (4.64 mmmol) in toluene (1.7 mL) was dropwise added to a suspension of α-methyl 3,5-bis(trifluoromethyl)benzylalcohol (3.87 mmmol), N-methyl-2-nitrobenzenesulfonylamide (3.87 mmmol), and triphenylphosphine (4.64 mmmol) in toluene (6ml) at an internal temperature of 10 to 19°C. After completion of the dropwise addition, the resulting solution was stirred for 1 hour, hexane (3 mL) was added thereto, and then the mixture was stirred for 1 hour. The precipitated solid was filtered and a mother liquid was concentrated under reduced pressure, to obtain a crude product. As a result of comparison analysis to a standard with an HPLC, it was confirmed that 1.57 g of (R)-N-methyl-N-[α-methyl-3,5-bis(trifluoromethyl)benzyl]-2-nitrobenzenesulfonylamide represented by the following formula:

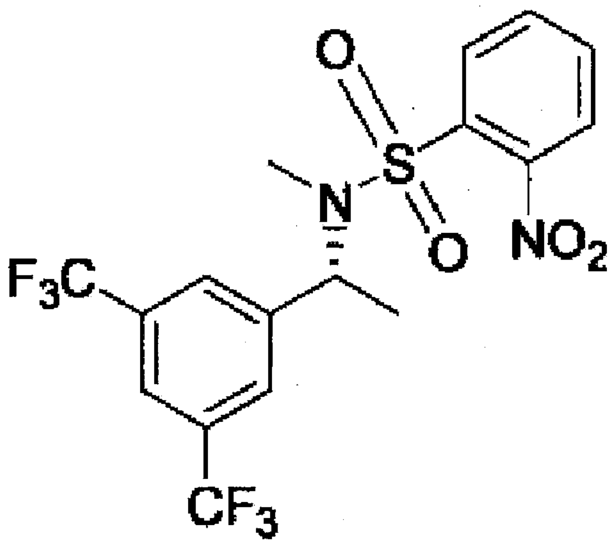

was contained (yield 89%). The obtained crude product was used in the next step without being purified further.
$^1$H-NMR(400MHz,$CDCl_3$) δ1.60 (3H,d), 2.73 (3H, s), 5.37-5.42 (1H,q), 7.66-7.79(6H,m), 8.05-8.07(1H,m)

(Example 2)

A 3M aqueous sodium hydroxide solution (38.58 mmol, 12.9 mL) was added to a toluene (20 mL) mixture containing (R)-N-methyl-N-[α-methyl-3,5-bis(trifluoromethyl)benzyl]-2-nitrobenzenesulfonylamide (12.95 mmol) obtained in Example 1, tetrabutylammonium bromide (1.30 mmol), and n-dodecanethiol (14.25 mmol), while controlling the internal temperature below 25°C. After completion of the addition, the resulting solution was stirred for 1 hour at an internal temperature 20 to 25°C. Water (10 mL) was added to the reaction mixture, and an organic phase was obtained with a liquid separation operation. pH in the system was adjusted to be 1.8 (at an internal temperature of 25°C) by adding water (30 mL) and adding concentrated hydrochloric acid to the obtained organic phase, and an aqueous phase was obtained with a liquid separation operation. pH in the system was adjusted to be 8.0 (at an internal temperature of 23°C) by adding ethyl acetate (30 mL) into the obtained aqueous phase and further adding a 30 wt% aqueous sodium hydroxide solution, and an organic phase was obtained with a liquid separation operation. The obtained organic phase was heated and concentrated under reduced pressure, to obtain 2.76 g of (R)-N-methyl-1-[3,5-bis(trifluoromethyl)phenyl]ethylamine. The yield was 79%. The optical purity of the crude product was 99.7%ee, the asymmetric transfer ratio by the 2 steps was 99.7%, and the substitution reaction with a nitrogen nucleophile proceeded with an inversion.
The asymmetric transfer ratio can be calculated with the following formula.

Asymmetric transfer ratio (%) = (Optical Purity (%ee) of Reaction Product / Optical Purity (%ee) of Reaction Substrate (Raw Material)) X 100

(Example 3)

A suspension containing (R)-N-methyl-N-[α-methyl-3,5-bis(trifluoromethyl)benzyl]-2-nitrobenzenesulfonylamide (0.66 mmol) obtained in Example 1, benzenethiol (0.79 mmol), potassium carbonate (1.98 mmol), and dimethylformamide (DMF) (3 mL) was stirred for 2 hours at an internal temperature of 24°C. Toluene (15 mL) and water (5 mL) were added to the reaction mixture, and an organic phase was obtained with a liquid separation operation. pH in the system was adjusted to be 1.7 (at an

internal temperature of 23°C) by adding water (10 mL) and adding concentrated hydrochloric acid to the obtained organic phase, and then an aqueous phase was obtained with a liquid separation operation. As a result of comparison analysis of the obtained aqueous phase to a standard with an HPLC, it was confirmed that 158 mg of (R)-N-methyl-1-[3,5-bis (trifluoromethyl)phenyl]ethylamine was obtained. The yield was 89%. The optical purity of the crude product was 99.5%ee, the asymmetric transfer ratio in the 2 steps was 99.5%, and the substitution reaction with a nitrogen nucleophile proceeded with an inversion.

(Example 4)

A solution of n-dodecanethiol (3.86 mmol) in DMSO (1 mL) was added to a suspension containing (R)-N-methyl-N-[α-methyl-3,5-bis(trifluoromethyl)benzyl]-2-nitrobenzenesulfonylamide (3.51 mmol) obtained in Example 1, lithium hydroxide-hydrate (10.52 mmol), and dimethylsulfoxide (DMSO) (3 mL) at an internal temperature of 27 to 31°C. After completion of the addition, the resulting mixture was stirred for 3 hours. Toluene (21 mL) and water (13 mL) were added to the reaction mixture, and an organic phase was obtained with a liquid separation operation. pH in the system was adjusted to be 1.9 (at an internal temperature of 23°C) by adding water (20 mL) and adding concentrated hydrochloric acid to the obtained organic phase, and then an aqueous phase was obtained with a liquid separation operation. As a result of comparison analysis of the obtained aqueous phase to a standard with an HPLC, it was confirmed that 857 mg of (R)-N-methyl-1-[3,5-bis(trifluoromethyl)phenyl]ethylamine was contained. The yield was 90%. The optical purity of the crude product was 99.5%ee, the asymmetric transfer ratio in the 2 steps was 99.5%, and the substitution reaction with a nitrogen nucleophile proceeded with an inversion.

(Example 5)

A solution of diisopropylazodicarboxylate (6.06 mmmol) in toluene (1.0 mL) was dropwise added to a suspension of (S)-1-phenyl-1-propanol (4.04 mmmol) (96.3%ee), N-benzyl-2-nitrobenzenesulfoneamide (4.45 mmmol), and triphenylphosphine (6.06 mmmol) in toluene (5 ml) while the internal temperature was kept at -3°C. After completion of the dropwise addition, the resulting mixture was stirred for 1 hour, hexane (2.5 mL) was added to the mixture, and the mixture was stirred further for 1 hour. The precipitated solid was filtered off and a mother liquid was concentrated under reduced pressure, to obtain a crude product. As a result of comparison analysis to a standard with an HPLC, it was confirmed that 1.55 g of (R)-N-benzyl-N-(1-phenylpropyl)-2-nitrobenzenesulfoneamide was contained (yield 94%). The obtained crude product was used in the next step without being purified further.
$^1$H-NMR(400MHz,$CDCl_3$) δ0.78(3H,t), 1.88-1.95(2H,m), 4.37(2H,dd), 4.97-5.00(1H,m), 7.12-7.62(14H,m)

(Example 6)

(R)-N-benzyl-N-(1-phenylpropyl)-2-nitrobenzenesulfone amide (3.79 mmol) obtained in Example 5, tetrabutylammonium bromide (0.38 mmol), a 1M aqueous sodium hydroxide solution (11.37 mmol, 11.4 mL), and toluene (12 mL) were mixed, and n-dodecanethiol (5.68 mmol) was added to the mixture at an internal temperature of 30 to 35°C. After completion of the addition, the resulting mixture was stirred for 4 hours at the same temperature, and an organic phase was obtained with a liquid separation operation. pH in the system was adjusted to be 1.7 (at an internal temperature of 27°C) by adding water (15 mL) and adding concentrated hydrochloric acid to the obtained organic phase, and an aqueous phase was obtained with a liquid separation operation. pH in the system was adjusted to be 9.7 (ataninternal temperature of 23°C) by adding a 30wt% aqueous sodium hydroxide solution to the obtained aqueous phase. Then, after the addition of ethyl acetate (20 mL), an organic phase was obtained with a liquid separation operation. The obtained organic phase was heated and concentrated under reduced pressure, to obtain 0.62 g of (R)-N-benzyl-1-phenylpropylamine. The yield was 64%. The optical purity of the product was 90.0%ee, the asymmetric transfer ratio in the 2 steps was 93.5%, and the substitution reaction by a nitrogen nucleophile proceeded with an inversion.
$^1$H-NMR(400MHz,$CDCl_3$) δ0.80(3H,t), 1.59-1.80(3H,m), 3.46-3.66(3H,m), 7.21-7.36(10H,m)

(Example 7)

A solution of diisopropylazocarboxylate (6.06 mmmol) in toluene (1. 0 mL) was dropwise added to a toluene (5 mL) suspension containing (S)-1,2-diphenylethanol (4.04 mmmol) (96.6%ee), N-ethyl-2-nitrobenzesulfoneamide (4.45 mmol), and triphenylphosphine (6.06 mmmol), while the internal temperature was kept at -3°C. After completion of the dropwise addition, the resulting mixture was stirred for 19 hours, and then water (10.0 mL) was added to the mixture. The reaction mixture was extracted with toluene (20.0 mL), and then was washed with a saturated sodium chloride

solution. The obtained extraction liquid was used in the next step without being purified further. As a result of comparison analysis to a standard with an HPLC, it was confirmed that 0.61 g of
(R)-N-ethyl-N-(1,2-diohenylethyl)-2-nitrobenzenesulfoneamid e was contained (yield 37%).
$^1$H-NMR(400MHz,$CDCl_3$) δ0.97(3H,t), 3.26-3.47(4H,m), 5.37-5.41(1H,m), 7.05-7.64(14H,m)

(Example 8)

n-dodecanethiol (2.23 mmol) was added to a suspension containing a toluene mixture (18 ml) of (R)-N-ethyl-N-(1,2-diphenylethyl)-2-nitrobenzenesulfoneamid e (1.49 mmol) obtained in Example 7, tetrabutylammonium bromide (0.15 mmol), and a 1M aqueous sodium hydroxide solution (4.47 mmol, 4.5 mL) at an internal temperature of 30 to 35°C. After completion of the addition, the resulting mixture was stirred for 18 hours at the same temperature, and an organic phase was obtained with a liquid separation operation. pH in the system was adjusted to be 0.6 (at an internal temperature of 27°C) by adding water (15 mL) and adding concentrated hydrochloric acid to the obtained organic phase. Thereafter, an aqueous phase was obtained with a liquid separation operation. pH in the system was adjusted to be 9.7 (at an internal temperature of 23°C) by adding a 30wt% aqueous sodium hydroxide solution to the obtained aqueous phase. Then, after the addition of ethyl acetate (20 mL), an organic phase was obtained with a liquid separation operation. The obtained organic phase was heated and concentrated under reduced pressure, to obtain 0.33 g of (R)-N-ethyl-1,2-diphenylethylamine. The yield was 83%. The optical purity of the product was 93.6%ee, the asymmetric transfer ratio in the 2 steps was 96.9%, and the substitution reaction by a nitrogen nucleophile proceeded with an inversion.
$^1$H-NMR(400MHz,$CDCl_3$) δ0.97(3H,d), 1.51(1H,br), 2.35-2.50(2H,m), 2.87-2.96(2H,m), 3.83-3.87(1H,m), 7.16-7.32 (10H,m)

## Claims

**1.** A method for producing an optically active benzylsulfonylamide derivative represented by a general formula (5):

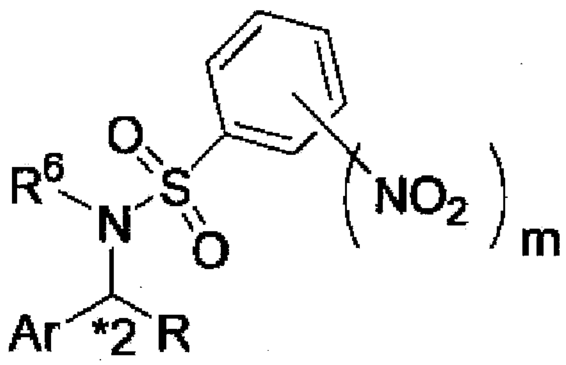

(5)

wherein Ar represents a substituted or unsubstituted aryl group having 6 to 20 carbon atoms or a substituted or unsubstituted heteroaromatic group having 4 to 20 carbon atoms, R represents a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, $R^6$ represents hydrogen, a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, m represents an integer of 1 to 3, and *2 represents an asymmetric carbon atom,
comprising reacting an optically active benzylalcohol derivative represented by a general formula (1):

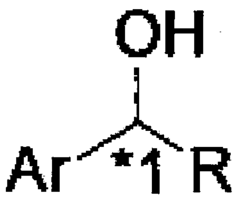

(1)

wherein Ar and R are as defined above, and *1 represents an asymmetric carbon atom, with a sulfonylamide derivative represented by a general formula (4):

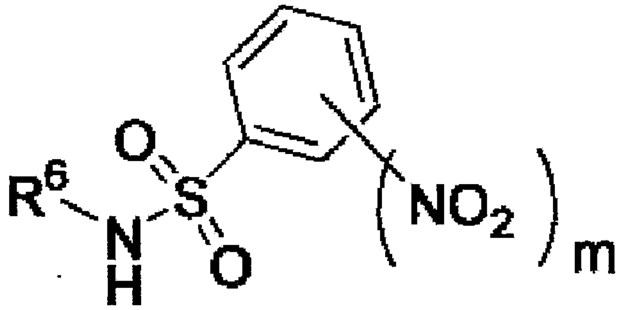

(4)

wherein $R^6$ and m are as defined above.

2. The production method according to Claim 1, wherein the reaction is performed in the presence of a phosphine derivative represented by a general formula (2):

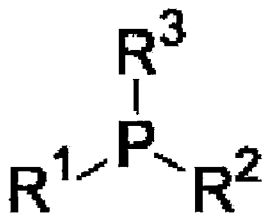

(2)

wherein $R^1$, $R^2$, and $R^3$ each independently represent a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, and an azodicarbonyl compound represented by a general formula (3):

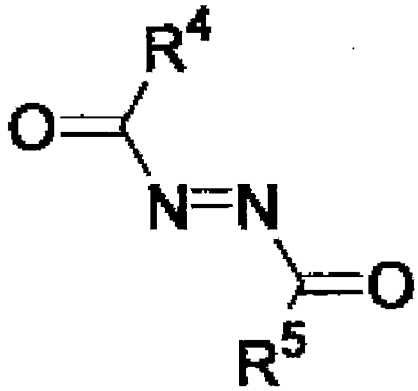

(3)

wherein $R^4$ and $R^5$ each independently represent a substituted or unsubstituted alkoxy group having 1 to 18 carbon atoms, a substituted or unsubstituted aryloxy group having 6 to 20 carbon atoms, or a substituted or unsubstituted aralkyloxy group having 7 to 20 carbon atoms.

**3.** A method for producing an optically active benzylamine derivative represented by a general formula (7):

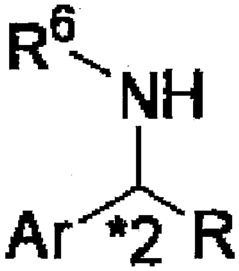

(7)

wherein Ar represents a substituted or unsubstituted aryl group having 6 to 20 carbon atoms or a substituted or unsubstituted heteroaromatic group having 4 to 20 carbon atoms, R represents a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, $R^6$ represents hydrogen, a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, and *2 represents an asymmetric carbon atom,
comprising reacting an optically active benzylsulfonylamide derivative represented by a general formula (5):

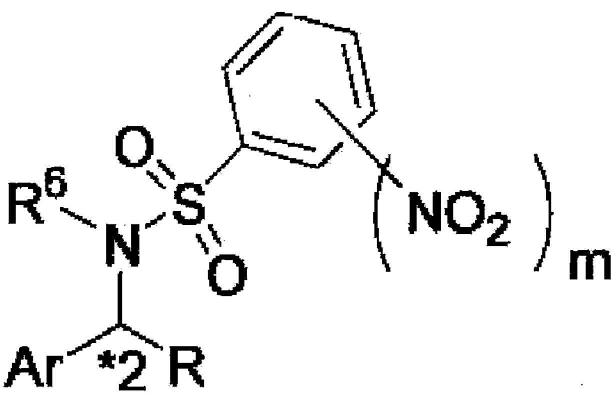

(5)

wherein Ar, R, $R^6$, and *2 are as defined above, and m represents an integer of 1 to 3, with a thiol derivative represented by a general formula (6):

$R^7SM$ (6),

wherein $R^7$ represents hydrogen, a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, and M represents hydrogen, an alkali metal, or an alkaline earth metal.

**4.** The production method according to Claim 3, wherein the reaction is performed under the coexistence of a base and/or a phase transfer catalyst.

**5.** The productionmethod according to Claim 3 or 4, wherein the optically active benzylsulfonylamide derivative represented by the formula (5) produced by the production method according to Claim 1 or 2 is used.

**6.** The production method according to any one of Claims 1 to 5, wherein Ar is a substituted or unsubstituted aryl group having 6 to 20 carbon atoms.

**7.** An optically active benzylsulfonylamide derivative represented by a general formula (5):

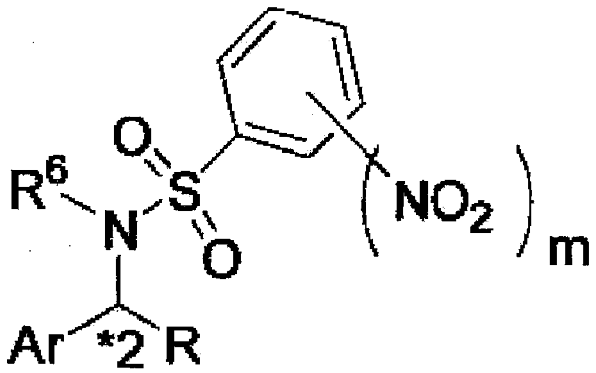

(5)

wherein Ar represents a substituted or unsubstituted aryl group having 6 to 20 carbon atoms or a substituted or unsubstituted heteroaromatic group having 4 to 20 carbon atoms, R represents a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, $R^6$ represents hydrogen, a substituted or unsubstituted alkyl group having 1 to 18 carbon atoms, a substituted or unsubstituted aryl group having 6 to 20 carbon atoms, or a substituted or unsubstituted aralkyl group having 7 to 20 carbon atoms, m represents an integer of 1 to 3, and *2 represents an asymmetric carbon atom.

**INTERNATIONAL SEARCH REPORT**

International application No.
PCT/JP2006/319106

A. CLASSIFICATION OF SUBJECT MATTER
*C07C303/40*(2006.01)i, *C07B53/00*(2006.01)i, *C07C209/62*(2006.01)i, *C07C211/27*(2006.01)i, *C07C311/16*(2006.01)i, *C07B61/00*(2006.01)n

According to International Patent Classification (IPC) or to both national classification and IPC

B. FIELDS SEARCHED

Minimum documentation searched (classification system followed by classification symbols)
C07C303/40, C07B53/00, C07C209/62, C07C211/27, C07C311/16, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | Christian A. O. et al., 'The Choice of Phosphane Reagent in Fukuyama-Mitsunobu Alkylation: Intramolecular Selectivity Between Primary and Secondary Alcohols in the Preparation of Asymmetric Tetraamine Building Blocks for Synthesis of Philanthotoxins' Eur.J.Org.Chem., 2003, No.17, p.3288-3299 | 1,2,7<br>1,2 |
| X<br>Y | Jiabing W. et al., 'Non-peptide $\alpha_v\beta_3$ antagonists. Part 7: 3-Substituted tetrahydro-[1,8]naphthyridine derivatives', Bioorg.Med.Chem.Lett., 2004, Vol.14, No.4, p.1049-1052 | 7<br>1,2 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 December, 2006 (08.12.06) | 26 December, 2006 (26.12.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

INTERNATIONAL SEARCH REPORT

International application No.
PCT/JP2006/319106

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | Minoru Y. et al., 'Dirhodium(II) tetrakis [N-tetrachlorophthaloyl-(S)-tertleucinate]: a new chiral Rh(II) catalyst for enantioselective amidation of C-H bonds' Tetrahedron Letters, 2002, Vol.43, No.52, p.9561-9564 | 7<br>1,2 |
| X<br>Y | I. B. Rozentsveig et al., 'Synthesis and Properties of N-(2,2,2-Trichloroethylidene)- and N-(2,2,2-Trichloroethyl) nitrobenzenesulfonamides', Russian Journal of Organic Chemistry, 2001, Vol.37, No.1, pages 87 to 92 | 7<br>1,2 |
| X<br>Y | JP 10-195038 A (Hokuriku Seiyaku Co., Ltd.), 28 July, 1998 (28.07.98), Full text & WO 1998/21177 A1 & EP 943606 A1 | 7<br>1,2 |
| Y | Remy C. L. et al., 'Quinazolinone-based fungal efflux pump inhibitors. Part 1: Discovery of an (N-methylpiperazine)-containing derivative with activity in clinically relevant Candida spp.' Bioorg. Med.Chem.Lett., 2004, Vol.14, No.20, p.5127-5131 | 1,2 |

Form PCT/ISA/210 (continuation of second sheet) (April 2005)

**INTERNATIONAL SEARCH REPORT**

International application No.
PCT/JP2006/319106

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1, 2, 7

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

INTERNATIONAL SEARCH REPORT

International application No.
PCT/JP2006/319106

Claims 1-7 of this international application are considered to contain the following two inventions having different special technical features.

(A) An optically active benzenesulfonylamide derivative represented by the general formula (5) and a production method thereof (claims 1, 2, 7)

(B) A method for producing an optically active benzylamine derivative represented by the general formula (7) (claims 3-6)

The inventions (A) and (B) relate to different compounds and their respective production methods.
In addition, the backbone common to the compounds of (A) and (B) has been known to a person skilled in the art as disclosed in documents D1-D5 listed below.

Since there is no technical feature common to all the claims 1-7, this international application does not satisfy the requirement of unity of invention.

Document D1: Christian A. O. et al.
'The Choice of Phosphane Reagent in Fukuyama-Mitsunobu Alkylation: Intramolecular Selectivity Between Primary and Secondary Alcohols in the Preparation of Asymmetric Tetraamine Building Blocks for Synthesis of Philanthotoxins'
Eur. J. Org. Chem., 2003, No.17, p.3288-3299

Document D2: Jiabing W. et al.
'Non-peptide $\alpha_v\beta_3$ antagonists. Part 7: 3-Substituted tetrahydro-[1,8]naphthyridine derivatives'
Bioorg. Med. Chem. Lett., 2004, Vol.14, No.4, p.1049-1052

Document D3: Minoru Y. et al.
'Dirhodium(II) tetrakis[N-tetrachlorophthaloyl-(S)-tert-leucinate]: a new chiral Rh(II) catalyst for enantioselective amidation of C-H bonds'
Tetrahedron Letters, 2002, Vol.43, No.52, p.9561-9564

Document D4: I. B. Rozentsveig et al.
'Synthesis and Properties of N-(2,2,2-Trichloroethylidene)- and N-(2,2,2-Trichloroethyl)nitrobenzenesulfonamides'
Russian Journal of Organic Chemistry, 2001, Vol.37, NO.1, p.87-92

Document D5: JP 10-195038 A (Hokuriku Seiyaku Co., Ltd.), 28 July, 1998 (28.07.98)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002030048 A


### Non-patent literature cited in the description


- *Journal of Organic Chemistry,* 1998, vol. 63, 6273
- *Journal of Organic Chemistry,* 1996, vol. 61, 3561
- *Tetrahedron Asymmetry,* 1996, vol. 7, 2809
- *Journal of Organic Chemistry,* 1990, vol. 55, 215
- *Tetrahedron Asymmetry,* 2003, vol. 14, 3581